# EUROPEAN PATENT SPECIFICATION

(11) **EP 2 282 638 B1**
(45) Date of publication and mention of the grant of the patent: **05.09.2012**
(21) Application number: 09745790.7
(22) Date of filing: 14.05.2009
(51) Int. Cl.: A01N 47/12

(54) **METHOD FOR IMPROVING THE TOLERANCE OF CROPS TO CHILLING TEMPERATURES AND/OR FROST**
VERFAHREN ZUR ERHÖHUNG DER TOLERANZ VON ZUCHTPFLANZEN GEGENÜBER KALTEN TEMPERATUREN UND/ODER FROST
PROCEDE POUR AMELIORER LA TOLERANCE DE CULTURES AUX TEMPERATURES FRAICHES ET/OU FROIDES

(30) Priority: 15.05.2008 EP 08356068; 11.06.2008 US 131665 P
(43) Date of publication of application: 16.02.2011
(73) Proprietor: Bayer CropScience AG, 40789 Monheim (DE)
(72) Inventor: MARTINEZ BARBOSA, Gustavo, C.P. 38030 (MX); TAILI, Lin, Fujian 352100 (CN); TAPIA RAMOS, Elias, Cuautitlan Izcalli 54750 (MX); VAN DEN EYNDE, Koen, 1785 Merchtem (BE); XUNFU, Zhang, Shandong 250100 (CN)
(74) Representative: Nowak, Alexander
(86) International application number: PCT/EP2009/055839
(87) International publication number: WO 2009/138458

(56) References cited:
- WO-A-2007/104660
- US-A- 3 992 185

## Description

The present invention relates to the use of propamocarb and derivatives thereof for improving the tolerance of crops to chilling temperatures and/or frost.

Temperature is one of the main factors, which affect the growth of crops. Chilling temperatures (of down to 0°C) and frost (temperatures of below 0°C) may slow down germination and crop growth and have a substantial effect on their development and on the quantity and quality of their products. Chill sensitive crop species suffer injury and/or substantially delayed development even at temperatures of below 5°C. Even temperatures which are slightly below 0°C may lead to partial or complete death of these crop species. Late frosts around the time of flowering, for example, repeatedly lead to substantial yield losses for example in pome and stone fruit species such as apple, pear, quince, peach, nectarine, apricot, plum, damson, almond or cherry. Crops, which have suffered chilling injury or frost damage, show dieback symptoms, for example on leaves, flowers and buds. Frost-damaged flowers develop no fruit at all or else deformed fruit or fruit with skin damage, which can only be sold with difficulty, if at all. Severe chilling injury and frost damage entails the death of the entire crop.

Chilling injury and frost damage are therefore important loss factors for the agricultural sector. Existing possibilities for avoiding chilling injury and frost damage are rather unsatisfactory owing to their complexity or the fact that the results are frequently not reproducible. Possibilities which must be mentioned in this context are the breeding of chill- and frost-resistant crop varieties, starting off chill-sensitive crops in the greenhouse and subsequently planting them out as late as possible, cultivation under plastic film, circulation of air in the stand, blowing in warm air, placing heaters in the stand, and irrigation frost protection.

WO 2007/104660 describes the use of compounds that inhibit the mitochondrial respiration chain at the level of the b/ci complex, in particular of active strobilurin compounds, for improving the tolerance of crops to chilling temperatures and/or frost.

DE 4437945 describes crop-strengthening products comprising vitamin E, which are said to reduce the crop-injurious effect of phytotoxic agrochemicals and other abiotic stressors. These compositions may additionally comprise cryoprotectants such as glycerol. The cryoprotectant, which is optionally present, is not described as having an effect, which prevents chilling injury or frost damage.

J. Lalk and K. Dorffling describe in Physiol. Crop. 63, 287-292 (1985) that abscisic acid can improve the frost resistance to chilling temperatures in hardened winter wheat.

It was an object of the present invention to provide compounds, which improve the tolerance of crops to chilling temperatures and/or frost.

This object has been achieved by using propamocarb or derivatives thereof for improving the tolerance of crops to chilling temperatures and/or frost.

Propamocarb is a known fungicidal active compound which is represented by the following formula I:

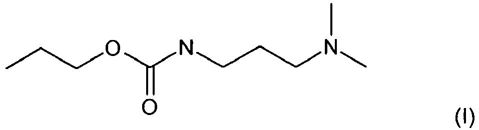

Propamocarb is described in British patent GB-1212708: activity is reported in controlling Pythium ultimum. This document is silent with regard to frost-tolerance.

Derivatives of propamocarb are exemplified by the following compounds:
(a) Propamocarb-hydrochloride or propamocarb-HCl which is a known compound having the chemical name propyl[3-(dimethylamino)propyl]carbamate hydrochloride and which is represented by the following formula la: 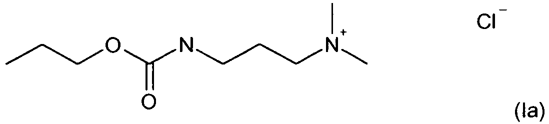
(b) Propamocarb-fosetylate or dimethyl-(propoxycarbonylamino)propyl]ammonium O-ethylphosphonate, is represented by the following formula Ib: 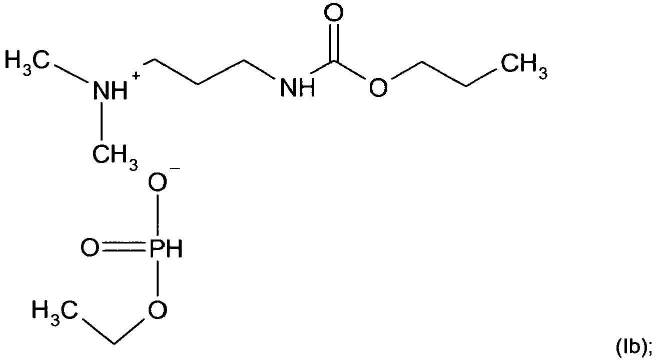
(c) Phosphoric acid derivatives of propamocarb, such as dimethyl-[3-(propoxycarbonylamino)propyl]-ammonium phosphate and dimethyl-[3-(propoxycarbonylamino)propyl]ammonium phosphate;
(d) all fungicidal active organic or inorganic acid salts of propamocarb.

According to the present invention, the cold stress that crops may undergo if a decrease in temperature occurs, can be prevented or effectively reduced by applying propamocarb or at least one of its derivatives.

In crop production, low temperatures are understood as meaning chilling temperatures and frost, i.e. temperatures below 15°C, preferably in the range of from 15°C to -15°C, especially preferably of from 10°C to -10°C and in particular of from 10°C to -5°C. Also temperature ranges of from 10°C to 0°C and 5°C to 0°C as well as temperature ranges of below 0°C can be damaging to the respective crop. Thereby, the temperature that leads to damages of crops may also depend on the crop concerned.

Propamocarb or at least one of its derivatives which are used in accordance with the invention are preferably employed for improving the tolerance of crops to a temperature range of from -15°C to 15°C, especially preferably of from -10°C to 10°C and in particular of from -5°C to 10°C. Furthermore, the improvement of the tolerance of crops to temperature ranges of from 10°C to 0°C, 5°C to 0°C as well as temperature ranges of below 0°C is specifically important.

In the case of chill-sensitive crops, propamocarb or at least one of its derivatives is employed in particular for improving the tolerance of the crops to chilling temperatures and to reduce the cold stress of crops in case of a decrease in temperature, respectively. This is generally understood as meaning a tolerance to temperatures in the range of from 0°C to 15°C, in particular of from 0°C to 10°C.

Frost-sensitive crops and/or chill-sensitive crops are, for example, selected from the group consisting of corn, soybean, rice, sugarbeet, potatoes, sugar cane, fruiting vegetables (tomatoes, bell pepper, chilli pepper, eggplant, cucumber, gherkins, melon, squash, pumpkin), leafy vegetables (lettuce, endive, spinach, brassica species) bean, pea, lentils, carrot, chicory, cabbage, flowers and ornamentals, banana, coffee and citrus species, pome and stone fruit. The pome fruit and stone fruit species are, for example, apple, pear, quince, peach, apricot, nectarine, cherry, plum, damson or almond, preferably apple. The citrus species are, for example, lemon, orange, grapefruit, clementine or tangerine.

In another embodiment of the invention the crops to be treated are transgenic crops.

Propamocarb or at least one of its derivatives are in particular also suitable for improving the tolerance of the crops to temperatures in the range of from -15°C to 0°C, especially preferably of from -10°C to 0°C, and in particular of from -5°C to 0°C.

Tolerance is understood as meaning in particular the reduction or prevention of chilling injury and/or frost damage in crops.

According to the present invention, cold stress is not restricted to frost damage by formation of ice crystals, but damages can also happen at temperatures above freezing-point, especially in sensitive crops. For such crops, even temperatures of for example 10°C to 5°C or 10°C to 0°C can result in significant damages. According to the present invention, it has been found that it is also possible to prevent sensitive crops from such damage by applying propamocarb or one of its derivatives according to the present invention.

In this manner, for example corn, soybean, rice, sugarbeet, potatoes, sugar cane, fruiting vegetables (tomatoes, bell pepper, chilli pepper, eggcrop, cucumber, gherkings, melon, squash, pumpkin), leafy vegetables (lettuce, endive, spinach, brassica species) bean, pea, lentils, carrot, chicory, cabbage, flowers and ornamentals, banana, coffee and citrus species (for example, lemon, orange, grapefruit, clementine or tangerine), pome and stone fruit (for example, apple, pear, quince, peach, apricot, nectarine, cherry, plum, damson or almond, preferably apple) can be effectively protected against cold stress or prevented from chilling injury.

According to the present invention propamocarb or at least one of its derivatives are employed in an application rate of from 25 to 1000 g/ha, particular preferably from 50 to 500 g/ha and in particular from 50 to 250 g/ha, based on the pure active ingredients.

According to the invention propamocarb or at least one of its derivatives may also be applied together with other active compounds, for example with herbicides, insecticides, growth regulators, fungicides or else with fertilizers.

When propamocarb or at least one of its derivatives or compositions comprising them, are combined with one or more further active compounds, in particular fungicides, it is in many cases possible, for example, to broaden the activity spectrum or to prevent the development of resistance. In many cases, synergistic effects are obtained.

Propamocarb or at least one of its derivatives are typically employed as formulations as they are conventionally used in the field of crop protection. Such formulations are commercially available, for instance, under the trade designations Previcur Energy^{®} (propamocarb-HCl + fosetyl-Al); Infinito^{®} (propamocarb-HCl + fluopicolide); Tattoo^{®} (propamocarb-HCl + mancozeb); Tattoo^{®}C, Lyos^{®}, Casoar^{®} (propamocarb-HCl + chlorothalonil) or Consento^{®} (propamocarb-HCl + fenamidone).

For example, they can be diluted with water in the form of concentrated solutions, suspensions or emulsions and applied by spraying. The use forms depend on the type of crop or the crop part to which it is to be applied; in any case, they should allow as fine as possible a distribution of the active compounds and auxiliaries.

In addition to propamocarb or at least one of its derivatives the formulations may comprise formulation auxiliaries as are conventionally used for the formulation of crop protection products, for example inert auxiliaries and/or surface-active substances such as emulsifiers, dispersants, wetters and the like.

Suitable surface-active substances are the alkali metal, alkaline earth metal and ammonium salts of aromatic sulfonic acids, for example lignosulfonic acid, phenol- sulfonic acid, naphthalenesulfonic acid and dibutylnaphthalenesulfonic acid and of fatty acids, alkyl- and alkylarylsulfonates, alkyl, lauryl ether and fatty alcohol sulfates, and the salts of sulfated hexa-, hepta- and octadecanols and of fatty alcohol glycol ethers, condensates of sulfonated naphthalene and its derivatives with formaldehyde, condensates of naphthalene or of the naphthalenesulfonic acids with phenol and formaldehyde, polyoxyethylene octylphenol ether, ethoxylated isooctyl-, octyl- or nonylphenol, alkylphenyl polyglycol ethers, tributylphenyl polyglycol ether, alkylaryl polyether alcohols, isotridecyl alcohol, fatty alcohol/ethylene oxide condensates, ethoxylated castor oil, polyoxyethylene or polyoxypropylene alkyl ethers, lauryl alcohol polyglycol ether acetate, sorbitol esters, lignosulfite waste liquors, methylcellulose or siloxanes. Examples of suitable siloxanes are polyether/polymethylsiloxane copolymers, which are also referred to as spreaders or penetrants.

Inert formulation auxiliaries are essentially: mineral oil fractions of medium to high boiling point such as kerosene and diesel oil, furthermore coal tar oils and oils of vegetable or animal origin, aliphatic, cyclic and aromatic hydrocarbons, for example paraffins, tetrahydronaphthalene, alkylated naphthalenes and their derivatives, alkylated benzenes and their derivatives, alcohols such as methanol, ethanol, propanol, butanol and cyclohexanol, ketones such as cyclohexanone, strongly polar solvents, for example amines such as N-methyl- pyrrolidone, and water.

Aqueous use forms of the compounds used according to the invention, specifically the compound I or at least one of its derivatives, can be prepared from storage formulations such as emulsion concentrates, suspensions, pastes, wettable powders or water-dispersible granules by addition of water. To prepare emulsions, pastes or oil dispersions, the compounds used according to the present invention, in particular the compounds of formula I or at least one of its derivatives as such or dissolved in an oil or solvent, can be homogenized in water by means of wetting agent, sticker, dispersant or emulsifier. Naturally, the use forms will comprise the auxiliaries used in the storage formulations.

In a preferred embodiment, propamocarb, its derivatives or formulations thereof are used in the form of an aqueous spray mixture. The aqueous spray mixture comprises propamocarb or at least one of its derivatives in an amount of preferably from 50 to 200 ppm.

In another embodiment the present invention relates to a method for improving the tolerance of crops to chilling temperatures and/or frost by treating the crops with propamocarb and or at least one of its derivatives.

The method according to the present invention can be employed for application in all of the abovementioned crops, but also in crop species, which differ from them. Depending on the crop part to which they are to be applied, they can be applied with apparatuses which are known per se and conventionally used in agricultural practice, application in the form of an aqueous spray solution or spray mixture being preferred.

The inventive method is suitable for foliar application in living crops or on the seedbed before transplanting, for soil applications prior to sowing or cropping, including overall soil treatment and furrow applications providing protection of the early stages of corn, wheat, soybean, cotton and other crops against chilling stress.

Application is effected by spraying to run-off point or by seed dressing. Either all of the aerial crop part or else only individual crop parts, such as flowers, leaves or fruits, are treated. The choice of the individual crop parts to be treated depends on the species of the crop and its developmental stage. Later stages may be protected preferably by leaf treatments. In one embodiment the application is onto seed. It is preferred to treat the embryos, seedlings, buds and flowers in various developmental stages, and the young fruits.

Application is preferably effected prior to a period of chilling temperature or frost. It is preferably effected at least 12 hours, especially preferably at least 24 hours and in particular 36 hours to 20 days before the expected onset of chilling temperatures or frost.

For treating seeds, in general the active compound is employed in amounts of from 1 to 1000 g/100 kg, preferably from 5 to 100 g/100 kg, of seed.

The present invention furthermore relates to a method for improving the tolerance of crops to low temperatures, preferably for reducing or preventing chilling injury and frost damage in crops, which comprises applying an aqueous composition comprising propamocarb or at least one of its derivatives used according to the invention, specifically a compound of formula I, to seed, crops or crop parts.

What has been said above with regard to the compounds used according to the invention, specifically the compounds of formula I, other components, the aqueous composition and the application, applies here analogously.

The tolerance of crops to chilling temperatures and frost is increased markedly by the use according to the invention of the active compound combinations described above. In particular, chilling injury and frost damage on crops are prevented or at least reduced by the use according to the invention.

Frost damage reduction based on the application of propamocarb and one of its derivatives based products can be evaluated under practical conditions in the open field. Increased frost tolerance in lettuce is achieved by applying Previcur Energy^{®} (propamocarb-HCl + fosetyl-Al)at a rate of 2.0 - 2.5 liter per ha and an application interval of 7- 10 days in a full grown crop. Frost tolerance can be observed after a night with temperatures below 0 C by assessing the percentage of damaged leaves. Damaged leaves are recognised by watery appearance on the day after the frost or by brown discolorisation at 3 days after frost. Previcur Energy will significantly reduce the frost damage compared to untreated.

In potatoes increased frost tolerance can be achieved by applying Infinito^{®} (propamocarb-HCl + fluopicolide) at a rate of 1.4 - 1.6 liter per ha at an application interval of 7- 10 days. Frost tolerance can be observed after the first night with temperatures below 0°C. Frost damaged leaves are recognized by watery appearance on the day after frost and these turn brown to black within a couple of days after frost. Infinito will significantly reduce frost tolerance compared to untreated.
Untreated:
frost damage 100% (all leaves and stems effected) yield 28.65 tons per ha
Treatment with Infinito:
frost damage 5% yield 38.64 tons per ha.

## Claims

1. Use of propamocarb or at least one of its derivatives for improving the tolerance of crops to chilling temperatures and/or frost, wherein propamocarb or the derivatives thereof are selected from the group consisting of p ropamocarb, propamocarb-HCl or propamocarb-fosetylate.

2. Use according to claim 1, wherein propamocarb or the derivatives are employed in mixtures with other fungicides and wherein such mixtures are selected from the group consisting of propamocarb-HCl and fosetyl-Al, propamocarb-HCl and fluopicolide, propamocarb-HCl and mancozeb, propamocarb-HCl and chlorothalonil, and propamocarb-HCl and fenamidone.

3. Use according to claims 1 or 2, wherein the crops to be treated are chill sensitive crops.

4. Use according to claim 3, wherein chill sensitive crops are selected from corn, soybean, rice, sugarbeet, potatoes, sugar cane, tomatoes, bell pepper, chili pepper, eggcrop, cucumber, gherkins, melon, squash, pumpkin, lettuce, endive, spinach, brassica species, beans, peas, lentils, carrot, chicory, cabbage, flowers and ornamentals, banana, coffee and citrus species, pome and stone fruit.

5. Use according to claim 4, wherein pome and stone fruit species are selected from apple, pear, quince, peach, apricot, nectarine, cherry, plum, damson or almond.

6. Use according to claim 4, wherein citrus species are selected from lemon, orange, grapefruit, clementine or tangerine.

7. Use according to claims I to 3, wherein the crops are transgenic crops.

8. Use according to claims 1 to 7, wherein propamocarb or at least one of its derivatives are applied to the crops to be treated with an application rate from 25 to 1000 g/ha.

9. Use according to claims 1 to 8, wherein propamocarb or at least one of its derivatives are used for improving the tolerance of crops to a temperature reange of from -15°C to 15°C.

10. Use according to claims 1 to 8, wherein propamocarb or at least one of its derivatives are used for improving the tolerance of crops to a temperature range of from -10°C to 10°C.

## Patentansprüche

1. Verwendung von Propamocarb oder mindestens einem seiner Derivate zum Verbessern der Kalttemperatur- und/oder Frosttoleranz von Kulturen, wobei Propamocarb oder seine Derivate aus der Gruppe bestehend aus Propamocarb, Propamocarb-HCl oder Propamocarb-Fosetylat ausgewählt werden.

2. Verwendung nach Anspruch 1, wobei Propamocarb oder die Derivate in Mischungen mit anderen Fungiziden eingesetzt werden und wobei solche Mischungen aus der Gruppe bestehend aus Propamocarb-HCl und Fosetyl-Al, Propamocarb-HCl und Fluopicolid, Propamocarb-HCl und Mancozeb, Propamocarb-HCl und Chlorothalonil sowie Propamocarb-HCl und Fenamidon ausgewählt werden.

3. Verwendung nach den Ansprüchen 1 oder 2, wobei es sich bei den zu behandelnde Kulturen um kälteempfindliche Kulturen handelt.

4. Verwendung nach Anspruch 1, wobei die kälteempfindlichen Kulturen aus der Reihe Mais, Sojabohne, Reis, Zuckerrübe, Kartoffeln, Zuckerrohr, Tomaten, Paprikaschoten, Chillipfeffer, Eierfrucht, Gurke, Einlegegurken, Melone, Sommerkürbis, Winterkürbis, Salat, Chicoree, Spinat, Brassica-Arten, Bohnen, Erbsen, Linsen, Karotte, Endivie, Kohl, Blumen und Zierpflanzen, Banane, Kaffee und Zitrusarten, Kern- und Steinobst ausgewählt werden.

5. Verwendung nach Anspruch 4, wobei die Kernobst- und Steinobstarten aus der Reihe Apfel, Birne, Quitte, Pfirsich, Aprikose, Nektarine, Kirsche, Pflaume, Damaszenerpflaume oder Mandel ausgewählt werden.

6. Verwendung nach Anspruch 4, wobei die Zitrusarten aus der Reihe Zitrone, Orange, Grapefruit, Klementine oder Mandarine ausgewählt werden.

7. Verwendung nach den Ansprüchen 1 bis 3, wobei es sich bei den Kulturen um transgene Kulturen handelt.

8. Verwendung nach den Ansprüchen 1 bis 7, wobei Propamocarb oder mindestens eines seiner Derivate auf die zu behandelnden Kulturen mit einer Aufwandmenge von 25 bis 1000 g/ha appliziert werden.

9. Verwendung nach den Ansprüchen 1 bis 8, wobei Propamocarb oder mindestens eines seiner Derivate zum Verbessern der Toleranz von Kulturen gegenüber einem Temperaturbereich von -15°C bis 15°C eingesetzt werden.

10. Verwendung nach den Ansprüchen 1 bis 8, wobei Propamocarb oder mindestens eines seiner Derivate zum Verbessern der Toleranz von Kulturen gegenüber einem Temperaturbereich von -10°C bis 10°C eingesetzt werden.

## Revendications

1. Utilisation du propamocarbe ou d'au moins l'un de ses dérivés pour améliorer la tolérance des cultures aux températures fraîches et/ou au gel, **caractérisée en ce que** le propamocarbe ou les dérivés de celui-ci sont choisis dans le groupe constitué par le propamocarbe, le popamocarbe-HCl ou le propamocarbe-fosétylate.

2. Utilisation selon la revendication 1, **caractérisée en ce que** le propamocarbe ou les dérivés sont utilisés en mélanges avec d'autres fongicides et **en ce que** de tels mélanges sont choisis dans le groupe constitué par le propamocarbe-HCl et le fosétyl-Al, le propamocarbe-HCl et le fluopicolide, le propamocarbe-HCl et le mancozèbe, le propamocarbe-HCl et le chlorothalonil, et le propamocarbe-HCl et la fénamidone.

3. Utilisation selon la revendication 1 ou 2, **caractérisée en ce que** les cultutres à traiter sont des cultures sensibles à la fraîcheur.

4. Utilisation selon la revendication 3, **caractérisée en ce que** les cultures sensibles à la fraîcheur sont choisies parmi le maïs, le soja, le riz, la betterave sucrée, les pommes de terre, la canne à sucre, les tomates, le poivron, le piment, l'aubergine, le concombre, le cornichon, le melon, la courge, la citrouille, la laitue, l'endive, l'épinard, les espèces brassica, les haricots, les pois, les lentilles, la carotte, la chicorée, le chou, les fleurs et les plantes ornementales, les espèces de bananes, de café et d'agrumes, les fruits à pépins et à noyau.

5. Utilisation selon la revendication 4, **caractérisée en ce que** les espèces de fruits à pépins et à noyaux sont choisies parmi la pomme, la poire, le coing, la pêche, l'abricot, la nectarine, la cerise, la prune, la quetsche ou l'amande.

6. utilisation selon la revendication 4, **caractérisée en ce que** les espèces d'agrumes sont choisies parmi le citron, l'orange, le pamplemousse, la clémentine ou la mandarine.

7. Utilisation selon les revendications 1 à 3, **caractérisée en ce que** les cultures sont des cultures transgéniques.

8. Utilisation selon les revendications 1 à 7, **caractérisée en ce que** le propamocarbe ou au moins l'un de ses dérivés sont appliqués aux cultures à traiter à un taux d'application de 25 à 1000 g/ha.

9. Utilisation selon les revendications 1 à 8, **caractérisée en ce que** le propamocarbe ou au moins l'un de ses dérivés sont utilisés pour améliorer la tolérance des cultures à une gamme de températures de -15°C à 15°C.

10. Utilisation selon les revendications 1 à 8, **caractérisée en ce que** le propamocarbe ou au moins l'un de ses dérivés sont utilisés pour améliorer la tolérance des cultures à une gamme de températures de -10°C à 10°C.
